(19) Europäisches Patentamt
European Patent Office
Office européen des brevets

(11) Publication number: **0 422 681 A1**

# (12) EUROPEAN PATENT APPLICATION

(21) Application number: **90119626.1**

(51) Int. Cl.[5]: **A61K 9/06**, A61K 47/42

(22) Date of filing: **12.10.90**

(30) Priority: **13.10.89 US 421421**

(43) Date of publication of application:
**17.04.91 Bulletin 91/16**

(84) Designated Contracting States:
**AT BE CH DE DK ES FR GB GR IT LI LU NL SE**

(71) Applicant: **SYNTEX (U.S.A.) INC.**
**3401 Hillview Avenue**
**Palo Alto California 94303(US)**

(72) Inventor: **Fu, Cherng-Chyi Roger**
**14050 Shadow Oaks Way**
**Saratoga, California 95070(US)**
Inventor: **Shek, Efraim**
**10700 Deep Cliff Drive**
**Cupertino, California 95014(US)**
Inventor: **Fleitman, Jeffrey S.**
**1594 Finch Way**
**Sunnyvale, California 94087(US)**
Inventor: **Leung, de Mei C.**
**1138 Jamestown Drive**
**Sunnyvale, California 94087(US)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**W-8000 München 40(DE)**

## (54) Collagen containing ophthalmic formulation.

(57) An ophthalmologically acceptable collagen containing aqueous composition is disclosed. The composition contains collagen and is a flowable liquid at temperatures below mammalian eye temperature (32° C to 42° C) and forms a gelled sustained release matrix after administration to the mammalian eye. The composition is comprised of ophthalmologically acceptable collagen material, a pharmaceutically active non-steroidal antiinflammatory drug, optionally an antibiotic, a buffer, a nonionic ethoxylated alkylphenol surfactant, a quaternary ammonium preservative, a tonicifier, a chelating agent, and optional excipients in an aqueous carrier. The collagen in the composition undergoes a change from a flowable liquid to a gelled matrix after the formulation is administered to the eye. The gelled matrix of the formulation traps and physically holds the drug in the matrix. When applied, the gel will remain in place in the cul-de-sac of the eye substantially longer than liquid formulations and will allow for a sustained release method of delivery of the drug to the eye. The drug release from the matrix and the drug half life are such that the formulation allows for once a day or even less frequent dosing which increases the convenience and improves patient compliance.

EP 0 422 681 A1

# COLLAGEN CONTAINING OPHTHALMIC FORMULATION

This invention relates to the field of collagen containing ophthalmic formulations and more particularly to such formulations which are flowable liquids at temperatures below mammalian eye temperature (32° C to 42° C) and form a gelled sustained release matrix after administration to the mammalian eye.

In general, an ophthalmic formulation contains an active compound and various ophthalmologically acceptable excipients, in the form of a solution, an ointment, a suspension, etc. An ophthalmologically acceptable excipient must be non-irritating to the eye and must not prevent the active ingredient from penetrating the blood-aqueous barrier and/or diffusing through the various ocular substructures to the site where it is pharmacologically active.

The excipients can include a tonicifier, a preservative, a surfactant, a buffering system, a chelating agent, a viscosity agent as well as other stabilizing agents. Ophthalmic formulations must be sterile, and if intended for multiple dosing regimens, must be preserved with an effective antimicrobial agent.

British Patent 1,556,097 published November 21, 1979, discloses sustained release ophthalmic pharmaceutical preparations containing collagens. The preparations include one or more ophthalmic drugs in a solubilized collagen or a collagen "fiber" dispersion. The formulation is applied to the eye as a collagen rod.

British Patent 1,386,864 published March 12, 1975, discloses micro-crystalline collagen-containing pharmaceutical compositions useful for topical applications. There are ophthalmic agents disclosed which are said to have prolonged duration when applied topically to the eye. The collagen thickens the composition to a gel which exhibits an essentially constant viscosity. The thickened composition, or gel, is applied to the eye.

US patent 4,713,446 describes a collagen solution with viscoelastic properties that becomes less viscous (more liquid) at physiological temperatures (32 to 48° C).

The American Journal of Ophthalmology 106 : 279-281 (Sept. 1988), describes the use of commercially available collagen corneal shields as ophthalmic sustained release drug delivery systems. These corneal shields are pre-manufactured and inserted in a manner similar to a contact lens.

A Science , 164 , 1282-1283 (June 1969) article describes the preparation of clear collagen gels for use as vitreous replacement. The gels of this article are injected into the interior of the eye.

European published application 306,984 discloses a stable, clear, antimicrobially effective, ophthalmic formulation comprising an ophthalmologically effective amount of a non-steroidal-antiinflammatory drug, and a preservative system formed of a quaternary ammonium preservative and a nonionic ethoxylated alkylphenol surfactant, and an aqueous vehicle. This application does not disclose either the use of collagen or a sustained release ophthalmic formulation.

Topical application of a drug to the eye in a conventional ophthalmic solution results in extensive drug loss. Current ophthalmic solutions are generally formulated as a conventional solution dosage form. The dosing regimen for such solutions calls for three to four applications per day. One approach that has been taken to improve ocular drug absorption and duration is to decrease the ocular drainage rate. This can be accomplished by use of a viscous vehicle such as an ointment or gel, etc. A preformed gel may give sustained release, however, the predetermined form may be uncomfortable since the eye must adjust to its presence. Such discomfort may lead to less patient compliance. There is still a need to develop a sustained release ophthalmic formulation for once a day dosing which is delivered to the eye as a flowable liquid, and forms a sustained release gel formulation after contact with the eye.

A primary object of the invention is to describe and disclose an aqueous ophthalmic formulation containing a non-steroidal antiinflammatory drug, and a collagen gelling agent. The advantage of this formulation is that it is a flowable liquid at temperatures below mammalian eye temperature (32° C to 42° C) and forms a sustained release soft gel after application to the eye. The sustained release nature of the gelled formulation means the formulation can be administered at a frequency of once a day or less.

Another object of the invention is to provide a formulation which is useful in the treatment of diseases of the eye or conditions associated with or accompanied by inflammatory processes, including, among others, glaucoma, cystoid macular edema, uveitis, diabetic retinopathy and conjunctivitis, or any trauma caused by eye surgery or eye injury and exhibits no adverse immunolgical or inflammatory effects.

Another object of this invention is an ophthalmic temperature sensitive gelable system for non-steroidal antiinflammatory drugs, said system comprising a collagen gelling agent in a concentration such that the system is a flowable liquid at temperatures below mammalian eye temperature (32° C to 42° C) and a gelled sustained release matrix after administration to the mammalian eye.

These and other objects of the present invention will become apparent to those persons skilled in the art upon reading the details of the composition, manufacture and usage as more fully set forth below.

It must be noted that as used in this specification and the appended claims, the singular forms "a", "an" and "the" include plural references unless the context clearly dictates otherwise. Thus, for example, reference to "a collagen gelling agent" includes mixtures of such agents, reference to "an NSAID" includes reference to mixtures of such NSAIDs, reference to "the method of administration" includes one or more different methods of administration known to those skilled in the art.

Definitions

As used herein, the term "NSAID" means an ophthalmologically acceptable non-steroidal anti-inflammatory drug including the isomers, esters, and pharmaceutically acceptable salts thereof. Examples of NSAIDs include ketorolac, indomethacin, flurbiprofen, diclofenac, and suprofen, including the isomers, esters, and pharmaceutically acceptable salts thereof. The most preferred NSAID is ketorolac tromethamine. "Ketorolac tromethamine" shall mean the compound (±)-5-benzoyl-1,2-dihydro-3H-pyrrolo[1,2-a]-pyrrole-1-carboxylic acid as the 2-amino-2-(hydroxymehtyl)-1,3- propanediol salt, also known as (±)-5-benzoyl-2,3-dihydro-1H-pyrrolizine-1-carboxylic acid with 2-amino-2-(hydroxymethyl)-1,3-propanediol (1:1) having the following structural formula

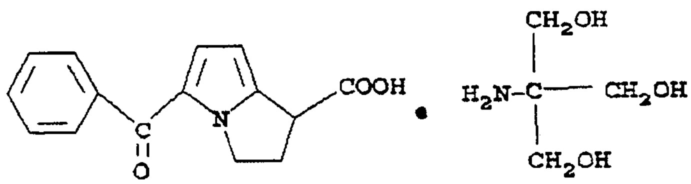

The NSAIDs do not have to be completely soluble in the formulation in order to be useful in the present invention.

As used herein, the term "collagen" means a hydroxyproline, glycine-type protein, the chief organic constituent of connective tissue and bones. Collagens useful in this invention are those which form a flowable liquid when present in NSAID ophthalmic formulations at temperatures below mammalian eye temperature (32°C to 42°C) and form a gel after the collagen containing formulation is applied to the mammalian eye. Two collagens which have been shown to be useful in this invention are Vitrogen 100® sold by Collagen Corporation of Palo Alto, California and Semed® S sold by Semex Medical of Frazer, Pennsylvania. Vitrogen 100® is sold as a sterile solution of purified, pepsin-solubilized bovine dermal collagen dissolved in 0.012N HCl. Semed® S is sold as a lyophilized collagen powder extracted from fresh bovine hides.

"Collagen gelling agent" is a temperature sensitive collagen material which will not affect the flowability of the ophthalmic formulation at a temperature below mammalian eye temperature (32°C to 42°C) but will cause the formulation to undergo a change to a sustained release gel after administration of the formulation to the eye. The collagen must (1) be ophthalmologically acceptable, (2) be stable over time in storage, and (3) not harmfully interact with the drug to be administered.

As used herein, the term "q.s." means adding a quantity sufficient to achieve a stated function, e.g., to bring a solution to the desired volume (i.e., 100%).

As used herein, the term "treatment" or "treating" means any treatment of a disease and/or condition in a mammal, including:

(i) preventing the disease and/or condition that is causing the clinical symptoms of the disease from developing;

(ii) inhibiting the disease and/or condition that is arresting the development of clinical symptoms; and/or

(iii) relieving the disease and/or condition that is causing the regression of clinical symptoms.

As used herein, the term "effective amount" means a dosage sufficient to provide treatment for the disease state being treated. This will vary depending on the patient, the disease and the treatment being effected.

As used herein, the term "antimicrobially effective" refers to the stability of the formulation prior to administration and means ability to withstand the U.S. Pharmacopeia antimicrobial challenge put by a panel of microbes.

As used herein, the term "stabilizing" means keeping a formulation relatively uniform in consistency and antimicrobially effective for its minimum reasonable shelf life, e.g., at least one year.

"Gel" is used herein to include solid and semisolid systems of at least one constituent, consisting of a

condensed mass enclosing, and interpenetrated by a liquid. The term encompasses a coherent gel matrix which is rich in liquid and as such is often referred to as a jelly and includes hydrogels wherein the liquid component of the gel is water.

## Formulations

The formulated compositions of the present invention include a non-steroidal antiinflammatory drug in an effective amount for ophthalmic treatment, a quaternary ammonium preservative, a stabilizing amount of a nonionic ethoxylated alkylphenol surfactant, a chelating agent, a tonicifier, a buffer, and other excipients such as a viscosity agent as well as other stabilizing agents in an aqueous vehicle, and a collagen gelling agent in such amount that the formulation is a flowable liquid at mammalian eye temperature or less, i.e. at <32° C and a gelled matrix after being administered to the mammalian eye. The final formulation must have the pH and ionic strength such that the collagen does not gel until after application of the formulation to the mammalian eye.

In another embodiment of the invention, the formulation includes as the ophthalmologically acceptable drug, an NSAID alone or combined with a second active agent each of which is present in an amount effective for ophthalmic treatment.

Ophthalmic solutions and suspensions typically contain an aqueous vehicle rather than an oily vehicle. Ophthalmic formulations must be sterile, and if intended for multiple dosing regimens, must be antimicrobially effective. The formulation is preferably antimicrobially effective for a minimum reasonable shelf life, e.g., at least six months, and preferably one to two years or more. The ingredients used in the formulations of the present invention are typically commercially available or can be made by methods readily known to those skilled in the art. Thus, preparation of formulations of the present invention will be possible by those skilled in the art upon reading this disclosure.

The ophthalmic formulations of this invention contain an effective amount, e.g., 0.001% to 10% wt/vol., preferably 0.001% to 5% wt/vol., most preferably 0.005% to 1% of a non-steroidal antiinflammatory drug. The amount of the non-steroidal antiinflammatory drug (NSAID) will vary with the particular formulation and the disease state for which it is intended. The total concentration of solutes should be such that, if possible, the resulting solution is isotonic with the lacrimal fluid (though this is not absolutely necessary) and has a pH in the range of 6 to 8.

The formulations of the present invention are prepared as solutions incorporating the above-described ingredients within the following approximate ranges:

| Ingredient | Amount |
|---|---|
| Collagen Gelling Agent | 0.01% to 20.0% wt/vol.; |
| Active Agent* | 0.001% to 10.0% wt/vol.; |
| Preservative | 0.001% to 1.0% wt/vol.; |
| Surfactant | 0.001% to 1.0% wt/vol.; |
| Buffer | 0.03% to 3.0% wt/vol.; |
| Tonicifier | 0.03% to 3.0% wt/vol; |
| Sodium Sulfate (anhydrous) | 0.07% to 7.0% wt/vol; |
| Other Excipients | 0% to 10.0% wt/vol.; |
| 1N NaOH or HC1 Solution | q.s. to adjust pH to 7.4±0.5; and |
| Purified Water | q.s. to 100%. |

*The active agent can be any pharmaceutically active drug such as an NSAID alone or in combination with another drug such as an antibiotic. The collagen containing aqueous based formulation of the present invention can provide for t e substained release of one or more drugs in a single formulation.

Optional excipients, such as a chelating agent, antioxidant, or viscosity agent, may be used. If a chelating agent is used, 0.01% to 1.0% wt/vol. is used. If an antioxidant is used, 0.05% to 1.0% wt/vol. is used. If a viscosity agent is used, 0.1% to 2.0% wt/vol. is used.

NSAIDs useful in the practice of this invention include, for example, ketorolac (and the other compounds described as being ophthalmologically effective in U.S. Patent No. 4,454,151 to Waterbury, issued

June 12, 1984, the pertinent portions of which are incorporated herein by reference), indomethacin, flurbiprofen, diclofenac, and suprofen, including the isomers, esters and pharmaceutically acceptable salts thereof.

Preservatives useful in the formulations of the present invention include quaternary ammonium compounds, such as cetyltrimethylammonium bromide, cetylpyridinium chloride and preferably, benzalkonium chloride (BAC).

Nonionic surfactants have been found to be useful in the formulations of the present invention. The surfactants of the present invention are ethoxylated alkylphenol surfactants of the following structure:

$RC_6H_4(OCH_2CH_2)_nOH$

wherein n has an average value of 30 to 50 and R is an alkyl of 7 to 10 carbon atoms. Useful ethoxylated alkylphenols include octoxynol 30, octoxynol 40, nonoxynol-30, nonoxynol-40, and nonoxynol-50. Octoxynol 40 is manufactured and sold by GAF under the tradename Igepal CA897® (a 70% aqueous solution of octoxynol 40), and is the most preferred nonionic ethoxylated alkylphenol surfactant.

The chelating agents useful in the formulations of the present invention include 8-hydroxyquinoline sulfate, citric acid, and preferably disodium edetate (EDTA $Na_2$). Under certain conditions, the chelating agent may also enhance the antimicrobial effect due to its ability to render essential metal ions unavailable to the microbes.

Buffering systems useful in the formulations of the present invention are based on, for example, citrate, borate, or phosphate.

Tonicifiers useful in the formulations of the present invention include dextrose, sorbitol, potassium chloride and/or sodium chloride.

Viscosity agents optionally useful in the formulations of the present invention include the cellulose derivatives such as hydroxypropylmethyl cellulose, sodium carboxymethylcellulose, and hydroxyethylcellulose.

Other optional excipients useful in the formulations of the present invention include stabilizing agents such as antioxidants, e.g., sodium metabisulfite and ascorbic acid, depending on the NSAID used.

In that the formulations of the invention form gels, the drugs need not be soluble in the formulation. An insoluble drug can still be released from the gelled matrix and delivered to the eye. When an insoluble drug is present, the liquid should be shaken before application to uniformly disperse the drug in the liquid. Soluble drugs are, of course, often used and such formulations are prepared by dissolving the solutes (e.g., the NSAID, the preservative, the surfactant, the chelating agent, and the buffering agent) in a suitable quantity of water, adjusting the pH to about 6 to 8, preferably 6.8 to 8.0 and most preferably 7.4, making a final volume adjustment to 100% with additional water, and sterilizing the preparation using any suitable method known to those skilled in the art, e.g., filtration. If the drug is not soluble, the drug is dispersed to the extent possible in the collagen and in some cases it may be possible to add other components to increase the solubility of the drug.

Ophthalmic formulations incorporating the preservative system of the invention are physically and functionally stable (i.e., remain antimicrobially effective) for at least the minimum reasonable shelf life of such products. The inclusion of other active drugs should be monitored to insure that the drug does not affect the rate of diffusion of the NSAID.

The type and amount of collagen material must be carefully selected in order to provide the best possible results for the formulations of the present invention. It is well known (e.g., Treatise on Collagen Volume 1, Chemistry of Collagen, edited by G. N. Ramachandran, 1967, Academic Press, and references therein) that the fluid or gel nature of a given collagen is dependent on the pH of the solution (increasing pH can gel or liquify a collagen, depending on the collagen), on the temperature of the solution (increasing the temperature may gel or liquify a collagen, again depending on the collagen), and the ionic strength (salt concentration) of a collagen solution (changing the ionic strength may gel or liquify a collagen, again depending on the collagen). The ordinary person skilled in the art will understand the factors that influence the collagen concentration, ionic strength, pH, etc. of a collagen solution in the context of the preparation of the gelable ophthalmic formulations of the present invention. The collagen material may be present in the formulation in an amount of about 0.01 to 20% wt/vol., preferably 0.1 to 5% wt/vol. For collagens such as Vitrogen 100®, the concentration is preferably 0.1 to 0.5% wt/vol. and most preferably about 0.2% wt/vol. For collagens such as Semed® S, the concentration is preferably 0.1 to 5% wt/vol, more preferably about 2% wt/vol. The collagen must be of such type and present in such amount as to allow the formulation to remain a flowable liquid at temperatures below mammalian eye temperature (32°C to 42°C) and then gel the formulation after being administered to the mammalian eye. The collagen provides the formulation with a temperature sensitivity (flowable liquid below mammalian eye temperature and gel after administration to the eye) while the solution (formulation) provides the collagen with the proper pH and ionic strength so that

the collagen remains a flowable liquid until after the formulation is administered to the eye.

Preferred Formulations

The preferred ophthalmic formulation of the invention includes a gelable collagen (flowable liquid at temperatures below mammalian eye temperature, undergoing a change to a gel after administration to the mammalian eye), an NSAID active agent in an effective amount for ophthalmic treatment, an antimicrobially effective amount of a quaternary ammonium preservative, an ethoxylated alkylphenol surfactant, a tonicifier, a chelating agent, and an aqueous vehicle.

The preferred collagen gelling agents that have been found to work particularly well in the present invention are sold commercially under the tradenames Vitrogen 100® (sold by Collagen Corporation of Palo Alto, California) and Semed® S (sold by Semex Medical of Frazer, Pennsylvania). If Semed® S is used the concentration of collagen used is higher, as shown in the below examples.

The preferred preservative of the invention is benzalkonium chloride (BAC).

The preferred surfactant of the invention is a nonionic ethoxylated alkylphenol surfactant, most preferably an octoxynol 40 sold under the tradename Igepal CA897®.

The preferred chelating agent of the invention is disodium edetate.

The preferred antibiotic is one which does not interfere with the corneal permeability of the NSAID. Tobramycin is a particularly preferred antibiotic.

The preferred ophthalmic solutions of the invention include an NSAID, a collagen gelling agent, benzalkonium chloride, octoxynol 40, disodium edetate, sodium chloride, sodium sulfate and sodium phosphate, and may include, as a second active agent, tobramycin. The preferred ophthalmic solutions must have the pH and ionic strength necessary to provide a flowable liquid formulation below mammalian eye temperature and a gel matrix after administration to the eye.

A preferred ophthalmic NSAID solution has the following formulation:

| Ingredient | Amount |
|---|---|
| Collagen Gelling Agent | 0.01% to 10% wt/vol.; |
| NSAID | 0.01% to 5% wt/vol.; |
| BAC (50% aq. soln.) | 0.01% to 0.5% wt/vol.; |
| Octoxynol 40 (70% aq. soln.) | 0.01% to 0.5% wt/vol.; |
| EDTA $Na_2$ | 0.05% to 1.0% wt/vol.; |
| Sodium Chloride | 0.03% to 3.0% wt/vol.; |
| Sodium Sulfate (anhydrous) | 0.07% to 7.0% wt/vol.; |
| Sodium Phosphate (dibasic) | 0.03% to 3.0% wt/vol.; |
| 1N NaOH or 1N HCl | q.s. to adjust pH to 7.4±0.5; and |
| Purified Water | q.s. to 100%. |

| Ingredient | Amount |
|---|---|
| Collagen Gelling Agent | 0.01% to 10% wt/vol.; |
| NSAID | 0.01% to 5% wt/vol.; |
| antibiotic | 0.01% to 5% wt/vol.; |
| BAC (50% aq. soln.) | 0.01% to 0.5% wt/vol.; |
| Octoxynol 40 (70% aq. soln.) | 0.01% to 0.5% wt/vol.; |
| EDTA $Na_2$ | 0.05% to 1.0% wt/vol.; |
| Sodium Chloride | 0.03% to 3.0% wt/vol.; |
| Sodium Sulfate (anhydrous) | 0.07% to 7.0% wt/vol.; |
| Sodium Phosphate (dibasic) | .03% to 3.0% wt/vol.; |
| 1N NaOH or 1N HCl | q.s. to adjust pH to 7.4±0.5; and |
| Purified water | q.s. to 100%. |

Most preferred is the ophthalmic solution according to the above formulations wherein the collagen gelling agent is of such a type and present in such amount that the formulation is a flowable liquid at less than mammalian eye temperature (i.e. 32°C or less), is readily dispensed in drops, and will undergo a change to a gel after administration to the mammalian eye (32°C to 42°C). The consistency of the gel allows it to conform to the shape of the eye cul-de-sac in which it is formed yet prevents it from being readily washed out of the eye with tears. The non-flowing consistency of the gel results in an excellent sustained release drug delivery system. Further, the collagen can be placed in contact with the eye and not exhibit adverse immunological or inflammatory effects.

## Utility and Administration

This invention is directed to collagen containing ophthalmic formulations with sustained release capability and methods useful for treating ophthalmic diseases in mammals. The NSAIDs of the formulations of this invention are used to treat diseases which are either caused by, associated with or accompanied by inflammatory processes, including, among others, glaucoma, cystoid macular edema, uveitis, diabetic retinopathy and conjunctivitis, or any trauma caused by eye surgery or eye injury.

The method of this invention is both curative and preventative. The gelled formulation formed after administration to the eye provides a matrix allowing for sustained release of the drug from the gel matrix to the eye. This sustained release feature allows for once-a-day dosing thus providing for greater patient compliance. When an NSAID is applied, for example, pre-surgically or immediately post-traumatically, i.e. before inflammation develops, it prevents development of inflammation. When an NSAID is applied directly to the eye suffering from any of the named ophthalmic diseases, it suppresses already developed inflammatory processes.

When the formulation of the invention includes an antibiotic such as tobramycin, the formulation also has antibacterial properties useful in eliminating and/or preventing a bacterial infection.

Ophthalmic solution formulations are typically administered by topical application to the eye or by instillation into the space (cul-de-sac) between the eyeball and the eyelids. The formulation of the invention is a flowable liquid at temperatures below mammalian eye temperature such that it can be readily dispensed from an eyedropper in drops to the eye and undergoes a change to a gel after administration to the eye. The gel matrix remains trapped in the eye cul-de-sac where the gel slowly dissolves and provides sustained release of the active drug or drugs from the gel to the eye.

The dosage level will, of course, depend on the NSAID or NSAIDs used, the concentration of the drops, the condition of the subject and the individual response to treatment. A typical dosage range might be about 2 to 10 drops, preferably 2 to 5 drops, of formulation containing active ingredient per day. If the formulation includes 0.5% wt/vol. of ketorolac tromethamine the amount of ketorolac tromethamine present is 0.05 to 0.25 mg, preferably 0.05 to 0.13 mg. If the formulation includes 0.3% wt/vol. of tobramycin the amount of tobramycin present is 0.03 to 0.15 mg, preferably 0.03 to 0.08 mg.

For a more detailed discussion of ophthalmic formulations in general, their preparation and administration, see Remington's Pharmaceutical Sciences , 15th Ed., pages 1489-1504, (1975).

Physical stability of the formulations of the present invention may be measured by preparing formulations, e.g., according to the foregoing Examples, sealing them in sterilized containers, and observing the formulation for any change in color, texture or light transmission after a period of one month and again after six months. Solutions that remain substantially unchanged would be considered stable.

Preservative efficacy of the formulations of the present invention is measured by preparing formulations, e.g., according to the below Examples, and subjecting them to the U.S. Pharmacopeia antimicrobial challenge.

The formulations of the present invention demonstrate preservative efficacy when tested in accordance with the above procedure.

## Testing

Ophthalmic formulations such as the solutions of the present invention are typically tested for physical stability, chemical stability, and preservative efficacy, both when they are first manufactured and after a fixed period of time (e.g., after two years). They are generally considered to be safe and clinically acceptable if proven to be well tolerated in the eye.

Physical stability is determined by observation of a solution after expiration of a fixed period of time. A

solution is considered to be physically stable if its appearance (e.g., color and clarity) does not change and if the pH remains constant, within acceptable limits. Chemical stability involves a routine chemical analysis of the solution, to be sure that its active ingredient(s), preservative(s) and the excipients have not changed after a fixed period of time.

Preservative efficacy of the formulation is tested by the procedure described in the U.S. Pharmacopeia, whereby a solution is challenged with a panel of microbes and a determination is made as to whether a given microbe survives in it.

## EXAMPLES

The following examples are given to enable those skilled in the art to more clearly understand and to practice the present invention. They should not be considered as a limitation on the scope of the invention, but merely as being illustrative and representative thereof.

## EXAMPLE 1

This example illustrates pharmaceutical formulations for ophthalmic administration and their preparation.

A. The NSAID is ketorolac tromethamine and the collagen gelling agent is Vitrogen 100®:

| Ingredient | Amount |
|---|---|
| Vitrogen 100® | 0.2% wt/vol.; |
| ketorolac tromethamine | 0.50% wt/vol.; |
| BAC (50% aq. soln.) | 0.02% wt/vol.; |
| Octoxynol 40 (70% aq. soln.) | 0.01% wt/vol.; |
| EDTA $Na_2$ | 0.10% wt/vol.; |
| Sodium Chloride | 0.3% wt/vol.; |
| Sodium Sulfate (anhydrous) | 0.7% wt/vol.; |
| Sodium Phosphate (dibasic) | 0.3% wt/vol.; |
| 1N NaOH or 1N HC1 Solution | q.s. to adjust pH to 7.4±0.5; and |
| purified water to make 100% volume | |

The above ingredients are mixed, adding purified water until they are dissolved. A stock solution containing 2.86 gm sodium phosphate dibasic, 2.98 gm sodium chloride and 7.1 gm sodium sulfate per 100 ml is prepared. Octoxynol, benzalkonium chloride and disodium edetate are dissolved in a solution containing one tenth of the target volume of the above salt solution and one tenth of the target volume of 1N sodium hydroxide solution. Eight tenths of the target volume of Vitrogen 100® solution is added to the above solution in an ice bath and mixed well. Ketorolac tromethamine is then added to the solution. The pH is adjusted to 7.4±0.5 and the balance of the formulation is made up with purified water, adding a quantity sufficient to make 100% volume. The solution is then sterilized using filtration.

B. Other NSAIDs, such as those described above, can be used as the active compound in the preparation of the formulation of this example. For example,

| Ingredient | Amount |
|---|---|
| Vitrogen 100® | 0.21% wt/vol.; |
| Flurbiprofen Sodium | 0.03% wt/vol.; |
| BAC (50% aq. soln.) | 0.02% wt/vol.; |
| Octoxynol 40 | 0.01% wt/vol.; |
| EDTA $Na_2$ | 0.10% wt/vol.; |
| Sodium Chloride | 0.3% wt/vol.; |
| Sodium Sulfate (anhydrous) | 0.7% wt/vol.; |
| Sodium Phosphate (dibasic) | 0.3% wt/vol.; |
| 1N NaOH or HC1 Solution | q.s. to adjust pH to 7.4±0.5; and |
| purified water to make 100 ml. | |

The above ingredients are mixed as described in part A. above.

C. Other collagen gelling agents can be used to prepare the formulations of this example. For example,

| Ingredient | Amount |
|---|---|
| Semed® S | 2.0% wt/vol.; |
| ketorolac tromethamine | 0.50% wt/vol.; |
| BAC (50% aq. soln.) | 0.02% wt/vol.; |
| Octoxynol 40 (70% aq. soln.) | 0.01% wt/vol.; |
| EDTA $Na_2$ | 0.10% wt/vol.; |
| Sodium Chloride | 0.3% wt/vol.; |
| Sodium Sulfate (anhydrous) | 0.7% wt/vol.; |
| Sodium Phosphate (dibasic) | 0.3% wt/vol.; |
| 1N NaOH or HC1 Solution | q.s. to adjust pH to 7.4±0.5; and |
| purified water to make 100% volume | |

The above ingredients are mixed as described in part A above.

EXAMPLE 2

This example illustrates pharmaceutical formulations for ophthalmic administration.

A. The NSAID is ketorolac tromethamine and the collagen gelling agent is Vitrogen 100®

| Ingredient | Amount |
|---|---|
| Vitrogen 100® | 0.2% wt/vol.; |
| ketorolac tromethamine | 0.50% wt/vol.; |
| BAC (50% aq. soln.) | 0.01% wt/vol.; |
| Octoxynol 40 (70% aq. soln.) | 0.02% wt/vol.; |
| EDTA $Na_2$ | 0.20% wt/vol.; |
| Sodium Chloride | 0.3% wt/vol.; |
| Sodium Sulfate (anhydrous) | 0.7% wt/vol.; |
| Sodium Phosphate (dibasic) | 0.3% wt/vol.; |
| 1N NaOH or HC1 Solution | q.s. to adjust pH to 7.4±0.5; and |
| purified water to make 100 ml. | |

The above ingredients are mixed as described in Example 1 A. above.

B. The NSAID is ketorolac tromethamine and the collagen gelling agent is Semed® S:

| Ingredient | Amount |
|---|---|
| Semed® S | 2.0% wt/vol.; |
| ketorolac tromethamine | 0.50% wt/vol.; |
| BAC (50% aq. soln.) | 0.01% wt/vol.; |
| Octoxynol 40 (70% aq. soln.) | 0.02% wt/vol.; |
| EDTA $Na_2$ | 0.20% wt/vol.; |
| Sodium Chloride | 0.3% wt/vol.; |
| Sodium Sulfate (anhydrous) | 0.7% wt/vol.; |
| Sodium Phosphate (dibasic) | 0.3% wt/vol.; |
| 1N NaOH or HC1 Solution | q.s. to adjust pH to 7.4±0.5; and |
| purified water to make 100 ml. | |

The above ingredients are mixed as described in Example 1 A. above.

C. Other NSAIDs, such as those described above, can be used as the active compound in the preparation of the formulations of this example.

## EXAMPLE 3

This example illustrates the preparation of pharmaceutical formulations for ophthalmic administration.

| A. Ingredient | Amount |
|---|---|
| Vitrogen 100® | 0.2% wt/vol.; |
| ketorolac tromethamine | 0.50% wt/vol.; |
| tobramycin | 0.30% wt/vol.; |
| BAC (50% aq. soln.) | 0.02% wt/vol.; |
| Octoxynol 40 (70% aq. soln.) | 0.01% wt/vol.; |
| EDTA $Na_2$ | 0.10% wt/vol.; |
| Sodium Chloride | 0.3% wt/vol.; |
| Sodium Sulfate (anhydrous) | 0.7% wt/vol.; |
| Sodium Phosphate (dibasic) | 0.3% wt/vol.; |
| 1N NaOH or HC1 Solution | q.s. to adjust pH to 7.4±0.5; and |
| purified water to make 100 ml. | |

The above ingredients are mixed as described in Example 1 A. above.

| B. Ingredient | Amount |
|---|---|
| Vitrogen 100® | 0.21% wt/vol.; |
| ketorolac tromethamine | 0.50% wt/vol.; |
| tobramycin | 0.30% wt/vol.; |
| BAC (50% aq. soln.) | 0.01% wt/vol.; |
| Octoxynol 40 (70% aq. soln.) | 0.01% wt/vol.; |
| EDTA $Na_2$ | 0.20% wt/vol.; |
| Sodium Chloride | 0.3% wt/vol.; |
| Sodium Sulfate (anhydrous) | 0.7% wt/vol.; |
| Sodium Phosphate (dibasic) | 0.3% wt/vol.; |
| 1N NaOH or HC1 Solution | q.s. to adjust pH to 7.4±0.5; and |
| purified water to make 100 ml. | |

The above ingredients are mixed as described in Example 1 A. above.

C. Other NSAIDs, and antibiotics can be used as the active compound(s) in the preparation of the formulation of this example.

D. Other collagen gelling agents, such as those described above, can be used in preparation of the formulations of this example.

## EXAMPLE 4

This example illustrates the preparation of pharmaceutical formulations for ophthalmic administration containing the NSAID ketorolac tromethamine.

| Ingredient | Amount |
|---|---|
| Vitrogen 100® | 0.21% wt/vol.; |
| ketorolac tromethamine | 2.0% wt/vol.; |
| BAC (50% aq. soln.) | 0.01% wt/vol.; |
| Octoxynol 40 (70% aq. soln.) | 0.02% wt/vol.; |
| EDTA $Na_2$ | 0.20% wt/vol.; |
| Sodium Chloride | 0.3% wt/vol.; |
| Sodium Sulfate (anhydrous) | 0.7% wt/vol.; |
| Sodium Phosphate (dibasic) | 0.3% wt/vol.; |
| 1N NaOH or HC1 Solution | q.s. to adjust pH to 7.4±0.5; and |
| purified water to make 100 ml. | |

The above ingredients are mixed as described in Example 1 A. above.

Other NSAIDs, such as those described above, can be used as the active compound in the preparation of the formulation of this example.

Other collagen gelling agents, such as those described above, can be used in the preparation of the formulation of this example.

## EXAMPLE 5

The effect of formulations of this invention on ocular inflammation resulting from topical sodium arachidonate

(NZW)SPF rabbits weighing between 2.3 and 5.0 kg were used. Six rabbits were used to evaluate each formulation. For each formulation 50 µl of formulation was applied to one eye, and a vehicle (50 µl) containing all components except the test agent was applied to the contralateral eye.

At a given time after drug administration, as shown in the below table, 50 µl of 0.5% sodium arachidonate was applied to both eyes. The sodium arachidonate solution was prepared just prior to its administration by the addition of 1.0 ml of 0.05 M sodium phosphate buffer (pH 7.4) to an ampoule containing 5 mg of sodium arachidonate (Sigma). The application of sodium arachidonate and the scoring of inflammation were at the times specified in the below table after the administration of the test formulation or vehicle.

The dosing of eyes was conducted on a random basis with the individual responsible for dosing not involved in scoring. The individual performing the scoring was unaware which of the eyes received the test formulation. The scoring procedure used was as follows:

| Lid Closure | Chemosis (conjunctival edema) |
|---|---|
| 0 eye open<br>1 1/3 closed<br>2 2/3 closed<br>3 completely closed | 0 none<br>1 slightly (barely discernable)<br>2 moderate (obvious edema)<br>3 severe (very marked edema) |

The data were analyzed using a paired-sample Wilcoxen signed-rank test to determine statistical significance.

| Formulation | Lid Closure Mean ± S.E. | Chemosis Mean ± S.E. |
|---|---|---|
| Vehicle | 3.0 ± 0.0 | 2.7 ± 0.2 |
| 0.5% KT*/S**, 4 hours | 1.3 ± 0.2[1] | 1.5 ± 0.2[1] |
| Vehicle | 3.0 ± 0.0 | 1.8 ± 0.3 |
| 0.5% KT*/S**, 24 hours | 1.2 ± 0.3[1] | 1.2 ± 0.3 |
| Vehicle | 2.7 ± 0.2 | 2.0 ± 0.3 |
| 0.5% KT*/V***, 24 hours | 1.5 ± 0.2 | 1.7 ± 0.2 |

[1]$p < 0.05$ 0f mean value compared with its corresponding vehicle/control mean, Wilcoxen statistic.

* KT = ketorolac tromethamine; formulation prepared according to Example 1.

** S = Semed® S collagen gelling agent

*** V = Vitrogen 100® collagen gelling agent

While the present invention has been described with reference to the specific embodiments thereof, it should be understood by those skilled in the art that various changes may be made and equivalents may be substituted without departing from the true spirit and scope of the invention.

## Claims

1. An ophthalmologically acceptable drug formulation which comprises a pharmaceutically acceptable non-steroidal antiinflammatory drug in an effective amount for ophthalmic treatment, a quaternary ammonium preservative, a stabilizing amount of a nonionic ethoxylated alkylphenol surfactant, a buffer, a tonicifier, a chelating agent, optional excipients, and an aqueous vehicle, **characterized by:** a collagen gelling agent, the collagen being present in such amount that the formulation is a flowable liquid at temperatures below mammalian eye temperature (32° C to 42° C) and undergoes a change to a sustained release gelled matrix after administration to the mammalian eye.

2. The ophthalmologically acceptable aqueous formulation of Claim 1 wherein the nonionic ethoxylated

alkylphenol surfactant is octoxynol 40.

3. The ophthalmologically acceptable aqueous formulation of Claim 1 or 2 wherein the quaternary ammonium preservative is benzalkonium chloride.

4. The ophthalmologically acceptable aqueous formulation of any one of Claims 1-3 wherein the pharmaceutically active non-steroidal antiinflammatory drug is selected from: ketorolac, indomethacin, flurbiprofen, diclofenac, and suprofen, and the isomers, esters, and pharmaceutically acceptable salts thereof.

5. The ophthalmologically acceptable aqueous formulation of Claim 4 wherein said non-steroidal antiinflammatory drug is ketorolac tromethamine.

6. The ophthalmologically acceptable aqueous formulation of any one of Claims 1-5 wherein the collagen gelling agent is selected from Vitrogen 100® and Semed® S.

7. The ophthalmologically acceptable aqueous formulation of Claim 6 wherein the collagen gelling agent is Vitrogen 100®.

8. An ophthalmologically acceptable aqueous sustained release formulation, comprising:

| | |
|---|---|
| NSAID | 0.001% to 10.0% wt/vol.; |
| Collagen gelling agent | 0.1 % to 20.0% wt/vol.; |
| Quaternary ammonium Preservative | 0.001% to 1.0% wt/vol.; |
| Nonionic ethoxylated alkylphenol Surfactant | 0.001% to 1.0% wt/vol.; |
| Disodium edetate | 0.05% to 0.2% wt/vol.; |
| Sodium Chloride | 0.03% top 3.0% wt/vol.; |
| Sodium Sulfate (anhydrous) | 0.07% to 7.0% wt/vol.; |
| Sodium Phosphate (dibasic) | 0.03% to 3.0% wt/vol.; |
| 1N NaOH or HC1 Solution | q.s. to adjust pH to 7.4±0.5; and |
| Purified Water q.s. to 100%. | |

9. The ophthalmologically acceptable aqueous sustained release formulation of Claim 8, wherein said collagen gelling agent is Vitrogen 100®.

10. The ophthalmologically acceptable aqueous sustained release formulation of Claim 9, comprising:

| | |
|---|---|
| ketorolac tromethamine | 0.50% wt/vol.; |
| Vitrogen 100® | 0.21% wt/vol.; |
| Benzalkonium chloride (50% aq. soln.) | 0.02% wt/vol.; |
| Octoxynol 40 (70% aq. soln.) | 0.01% wt/vol.; |
| Disodium edetate | 0.10% wt/vol.; |
| Sodium Chloride | 0.3% wt/vol.; |
| Sodium Sulfate (anhydrous) | 0.7% wt/vol.; |
| Sodium Phosphate (dibasic) | 0.3% wt/vol.; |
| 1N NaOH or HC1 Solution | q.s. to adjust pH to 7.4±0.5; and |
| purified water to make 100 ml. | |

11. The ophthalmologically acceptable aqueous sustained release formulation of Claim 9, comprising:

| | |
|---|---|
| ketorolac tromethamine | 0.50% wt/vol.; |
| Vitrogen 100® | 0.21% wt/vol.; |
| Benzalkonium chloride (50% aq. soln.) | 0.02% wt/vol.; |
| Octoxynol 40 (70% aq. soln.) | 0.01% wt/vol.; |
| Disodium edetate | 0.10% wt/vol.; |
| Sodium Chloride | 0.3% wt/vol.; |
| Sodium Sulfate (anhydrous) | 0.71% wt/vol.; |
| Sodium Phosphate (dibasic) | 0.286% wt/vol.; |
| 1N NaOH or HCl Solution | q.s. to adjust pH to 7.4±0.5; and |
| Purified Water q.s. to 100% volume. | |

12. An ophthalmic temperature sensitive gelable system for non-steroidal antiinflammatory drugs, said system comprising a collagen gelling agent in a concentration such that the system is a flowable liquid at temperatures below mammalian eye temperature (32°C to 42°C) and a gelled sustained release matrix after administration to the mammalian eye, a quaternary ammonium preservative, a buffer, a tonicifier, a chelating agent, optional excipients, and a stabilizing amount of a nonionic ethoxylated alkylphenol surfactant.

13. Use of a collagen gelling agent for the manufacture of a sustained release ophthalmic formulation containing a non-steroidal antiinflammatory drug for use in the treatment of diseases of the eye, the collagen being present in such amount that the formulation is a flowable liquid at temperatures below mammalian eye temperature (32°C to 42°C) and undergoes a change to a gelled sustained release matrix after administration to the eye.

European Patent Office

# EUROPEAN SEARCH REPORT

Application Number

EP 90 11 9626

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 126 684 (MERCK & CO. INC.)<br>* Abstract; page 3, lines 14-28; page 4, line 5 - page 5, line 29; claims 1-3 * | 1-13 | A 61 K 9/06<br>A 61 K 47/42 |
| Y | WO-A-8 400 548 (BATTELLE DEVELOPMENT CORP.)<br>* Page 1, line 25 - page 2, line 16; page 3, lines 3-6,23-30; page 7, lines 17-18; page 15, table II; claims 1,5 * | 1-13 | |
| Y | INVEST. OPHTHALMOL. VISUAL SCI., vol. 26, June 1985, pages 901-905; H.S. GEGGEL et al.: "Collagen gel for ocular surface"<br>* Whole document * | 1-13 | |
| Y | DERWENT FILE SUPPLIER WPI, 1975, accession no. 75-53113W [32], Derwent Publications, Ltd, London, GB; & JP-A-50 042 025 (JAPAN LEATHER IND.)<br>* Abstract * | 1-13 | |
| D,Y | EP-A-0 306 984 (SYNTEX INC.)<br>* Abstract; page 6, lines 6-44; examples; claims * | 1-13 | |
| A | ISRAEL. J. MED. SCI., vol. 3, no. 5, 1967, pages 755-758; S. SHOSHAN: "Cell growth promoting effect of enriched collagen solutions thermally gelled in vivo"<br>* Whole document * | 1-13 | **TECHNICAL FIELDS SEARCHED (Int. Cl.5)**<br>A 61 K |
| D,A | DE-A-2 719 770 (NIPPI INC.)<br>* Page 7, line 13 - page 8, line 7; page 13, lines 12-17; claims 1,2,7<br>* & GB-A-1 556 097 | 1-13 | |
| A | FR-A-2 173 736 (AVICON INC.)<br>* Page 1, line 1 - page 2, line 10; claims * | 1-13 | |

The present search report has been drawn up for all claims

| Place of search | Date of completion of search | Examiner |
|---|---|---|
| The Hague | 18 January 91 | HOFF P.J.L. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document